# EUROPÄISCHE PATENTSCHRIFT

(11) **EP 1 733 705 B1**
(45) Veröffentlichungstag und Bekanntmachung des Hinweises auf die Patenterteilung: **15.09.2010**
(21) Anmeldenummer: 06005941.7
(22) Anmeldetag: 23.03.2006
(51) Int. Cl.: A61F 2/34

(54) **Hüftgelenk Prothese**
Hip Prosthesis
Prothèse de Hanche

(30) Priorität: 14.06.2005 DE 102005027427
(43) Veröffentlichungstag der Anmeldung: 20.12.2006
(73) Patentinhaber: Mathys AG Bettlach, 2544 Bettlach (CH)
(72) Erfinder: Oberbach, Thomas, D-07629 Reichenbach (DE); Salomon, Dirk, D-04626 Jonaswalde (DE)
(74) Vertreter: Körfer, Thomas

(56) Entgegenhaltungen:
- EP-A- 0 169 978
- EP-A- 0 562 782
- EP-A- 0 694 294
- EP-A- 0 773 007
- EP-A- 0 803 234
- WO-A-00/64383
- WO-A-03/092557
- WO-A-2004/021934
- DE-A- 19 640 745
- DE-A- 19 813 074

## Beschreibung

Die Erfindung betrifft eine Hüftgelenk-Endoprothese gemäß dem Oberbegriff des Anspruchs 1 zur prothetischen Versorgung des menschlichen oder tierischen Hüftgelenks mit einer modularen Gelenkpfanne.

Es ist beispielsweise aus der EP 0 169 978 A1 eine Hüftgelenkspfanne bekannt, welche eine Außenschale und einen inneren Pfannenkörper umfaßt, wobei der Pfannenkörper mit einem konischen Mantel und die Außenschale mit einem an den Mantel angepaßten Hohlraum versehen sind.

Nachteilig an der aus der EP 0 169 978 A1 bekannten Hüftgelenkspfanne ist insbesondere, daß bei Ermüdungsbrüchen bei Belastungsspitzen bzw. bei Abnutzung des Pfannenkörpers die gesamte Hüftgelenkspfanne ersetzt werden muß, was einerseits einen beträchtlichen Aufwand mit langer Operationsdauer und einem erheblichen Materialabtrag im Bereich des Beckenknochens nach sich zieht und andererseits hohe Kosten verursacht.

Eine Hüftgelenk-Endoprothese gemäß dem Oberbegriff des Anspruchs 1 ist zum Beispiel in der WO 03/092557 A2 und der EP 0 803 234 A1 beschrieben. Aus WO 03/092557 A2 ist ein Inlay für eine Gelenkpfanne zu entnehmen, das mit einer aufsitzenden Ausgleichshülse zweiteilig ausgebildet ist, wobei das Inlay mit einem Innenteil ausgebildet sein kann, das jeweils eine exzentrisch angeordnete Gelenkkugel-Ausnehmung aufweist.

EP 0 803 234 A1 zeigt in mehreren Ausführungsbeispielen jeweils ein Inlay für eine Hüftgelenk-Endoprothese, das mit einer inneren Gelenkschale zweiteilig oder auch mit einer Außenschale dreiteilig ausgebildet sein kann.

Der Erfindung liegt demnach die Aufgabe zugrunde, eine Hüftgelenk-Endoprothese mit einer Gelenkpfanne zu schaffen, welche so ausgebildet ist, dass sie im Schadensfall in einfacher Weise revidiert werden kann, ohne die gesamte Gelenkpfanne aus dem Beckenknochen entfernen zu müssen, die bessere Eigenschaften bzgl. Haltbarkeit und Tragekomfort aufweist sowie Verformungen am Grundkörper der Hüft-Endoprothese ausgleicht.

Die Aufgabe wird durch die kennzeichnenden Merkmale des Anspruchs 1 gelöst.

Gemäß Anspruch 1 ist somit vorgesehen, den Grundkörper der Gelenkpfanne im Beckenknochen zu belassen und lediglich das Inlay auszutauschen. Unter Zwischenlage einer Ausgleichshülse ist es möglich, Verformungen des Grundkörpers in einfacher und kostengünstiger Weise auszugleichen. Die Operationsdauer wird dadurch signifikant verkürzt, die Risiken für den Patienten minimiert und die Kosten gesenkt.

Weitere vorteilhafte Ausgestaltungen der Erfindung sind in den Unteransprüchen gekennzeichnet.

Vorteilhafterweise ist das Inlay aus einem keramischen Werkstoff hergestellt, der eine Gleitpaarung mit einer ebenfalls keramischen Gelenkkugel bildet.

Weiterhin ist von Vorteil, daß die Ausgleichshülse vollflächig an dem Grundkörper und dem Inlay anliegt und so eine sichere Fixierung des Inlays im Grundkörper ermöglicht.

Ein Ausführungsbeispiel der Erfindung wird im folgenden anhand teilweise schematischer Darstellungen in der Zeichnung näher erläutert. In der Zeichnung zeigen:
- Fig. 1: eine schematische Ansicht eines Ausführungsbeispiels einer mit einer erfindungsgemäß ausgestalteten Gelenkpfanne ausgestattete Hüftgelenk-Endoprothese,
- Fig. 2A-B: Ansichten der Gelenkpfanne mit einem zu revidierenden Inlay sowie das aus der Gelenkpfanne ausgelöste zu revidierende Inlay der erfindungsgemäß ausgestalteten Gelenkpfanne der Hüftgelenk-Endoprothese gemäß Fig. 1, und
- Fig. 3A-B: Ansichten eines revidierten Inlays für eine erfindungsgemäß ausgestaltete Gelenkpfanne vor und nach dem Einsetzen in die Gelenkpfanne.

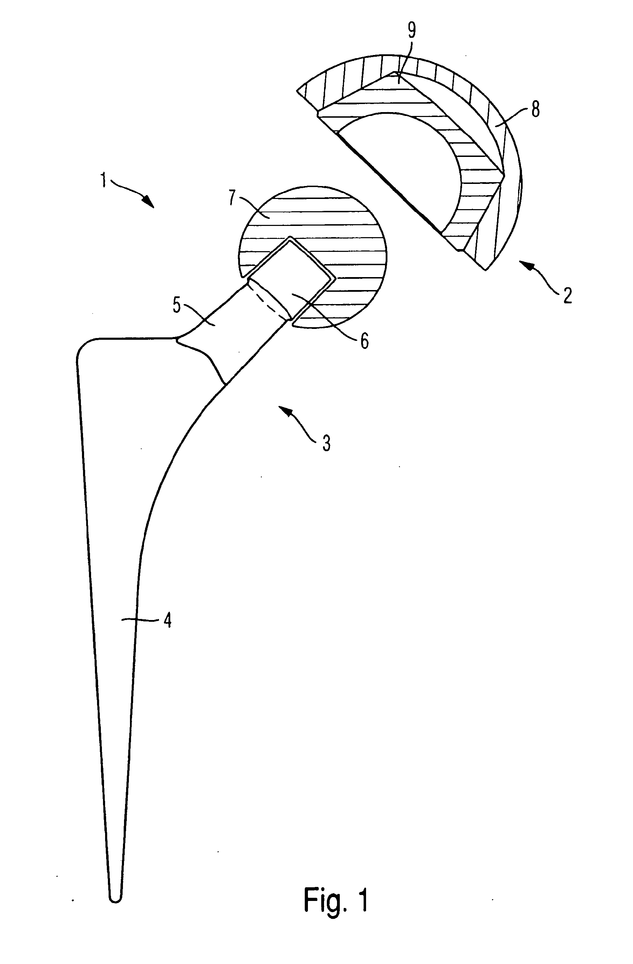

Fig. 1 zeigt in einer schematischen Darstellung eine Gesamtansicht einer Hüftgelenk-Endoprothese 1, die mit einer erfindungsgemäß ausgestalteten modularen Gelenkpfanne 2 kombinierbar ist. Die im Ausführungsbeispiel dargestellte Hüftgelenk-Endoprothese 1 wird auch als totale Hüftgelenk-Endoprothese 1 bezeichnet, da sowohl der femurale als auch der beckenseitige Gelenkanteil ersetzt wird. Eine Femurkomponente 3 weist einen Schaft 4 auf, welcher in der Markhöhle eines Femurknochens je nach Bauform der Femurkomponente 3 einzementiert oder zementfrei verankert wird. An dem Schaft 4 ist eine Halspartie 5 ausgebildet, an deren Ende ein Hüftschaftkonus 6 zur Aufnahme einer Gelenkkugel 7 ausgebildet ist.

Die Femurkomponente 3 ist nur sehr schematisiert dargestellt und kann auch in beliebigen anderen Ausführungsformen ausgebildet sein, sofern eine Gelenkkugel 7 vorgesehen ist. Die im folgenden näher beschriebenen erfindungsgemäßen Maßnahmen sind somit unabhängig von der Form der Femurkomponente 3 zu sehen.

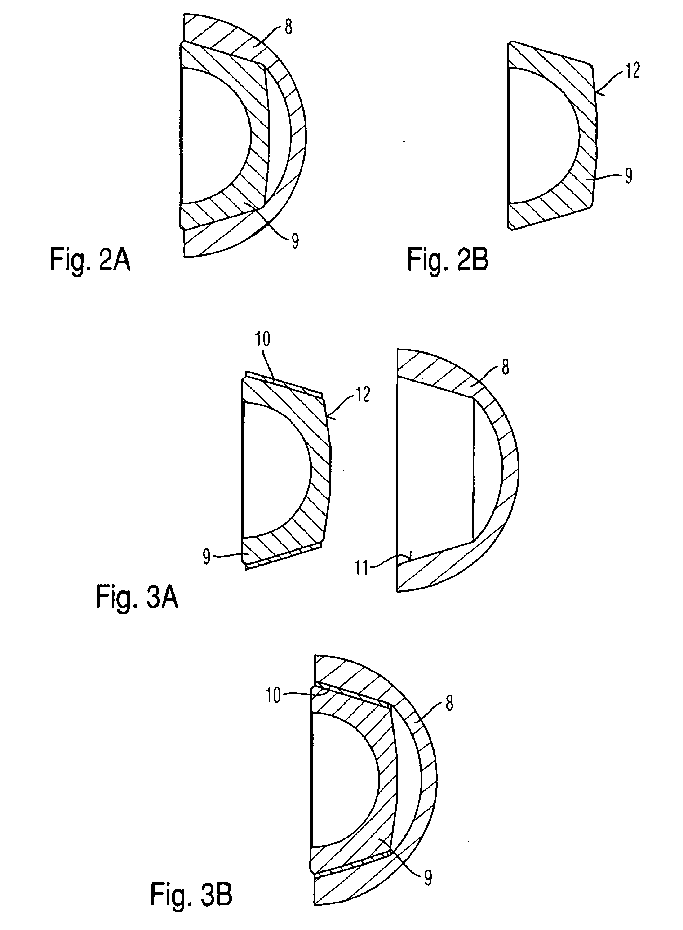

Die Gelenkkugel 7 liegt gleitend in einer modularen Gelenkpfanne 2, welche im folgenden unter Bezugnahme auf die Fig. 2A bis 2B und 3A bis 3B näher beschrieben wird.

Eine erfindungsgemäß ausgestaltete Gelenkpfanne 2 ist dabei aus zwei Komponenten aufgebaut, welche lösbar miteinander verbindbar sind. Ein vorzugsweise metallischer Grundkörper 8 der Gelenkpfanne 2 wird dabei beckenseitig im Knochen verankert. Dies kann beispielsweise bei geeigneter Ausgestaltung der Gelenkpfanne 2, wie im Ausführungsbeispiel dargestellt, durch Einzementieren oder auch bei passender Ausgestaltung zementfrei erfolgen.

In den Grundkörper 8 der Gelenkpfanne 2 ist, wie aus Fig. 2A ersichtlich, ein Inlay (ein Innenkörper) 9 eingelegt oder eingepreßt, welches vorzugsweise aus Keramik gefertigt ist und den Gleitpartner zu der in Fig. 1 dargestellten Gelenkkugel 7 darstellt. Als Gleitpaarungen bieten sich hier beispielsweise Keramik mit Keramik an.

In Hüftgelenk-Endoprothesen 1 kommt es immer wieder zu Revisionsfällen durch Abnutzung oder Bruch einzelner Komponenten, insbesondere der Gelenkkugel 7 und der Gelenkpfanne 2. Dies wird beispielsweise durch Abrieb zwischen den Gleitpartnern, das Einwandern von Abrieb oder Drittkörpern in den Gelenkspalt, Ermüdungsbrüche oder Gewaltbrüche bei Belastungsspitzen verursacht.

Um hier in einfacher Weise Abhilfe zu schaffen, die Operationsdauer möglichst kurz zu halten und den Knochenabtrag im Bereich des Beckens nicht unnötig zu erhöhen, ist daher vorgesehen, den Grundkörper 8 in situ zu belassen, das schadhafte Inlay 9 zu entfernen und durch ein neues Inlay 9 unter Zwischenlage einer Ausgleichshülse 10 einzulegen.

Fig. 2B zeigt das revisionsbedürftige Inlay 9, welches bereits aus dem Grundkörper 8 der Gelenkpfanne 2 herausgelöst ist. Anstelle des schadhaften Inlays 9 wird, wie in Fig. 3A und 3B dargestellt, ein nächstkleineres Inlay 9, welches jedoch für den gleichen Durchmesser der Gelenkkugel 7 ausgelegt ist, unter Zwischenlage einer Ausgleichshülse 10 in den Grundkörper 8 eingebracht. Die Ausgleichshülse 10 ist dabei aus dem biokompatiblen Polymer Polyetheretherketon (PEEK) oder Polymethylmethacrylat (PMMA) oder aus einem Verbundwerkstoff, gegebenenfalls auch mit geeigneten Beschichtungen, hergestellt. Sie dient dem Ausgleich von Verformungen und/oder leichten Beschädigungen des Konussitz des Grundkörpers 8 der Gelenkpfanne 2. Die Ausgleichshülse 10 legt sich nach dem Einsetzen des Inlays 9 an die innere Form des Konussitzes am Grundkörper 8 an und kompensiert geometrische Abweichungen von der Idealgeometrie des Konussitzes. Das Inlay 9 wird im Grundkörper 8 zentriert, wodurch eine großflächige, gleichmäßige Krafteinleitung in das Inlay 9 erfolgt.

Die Ausgleichshülse 10 liegt dabei vollflächig an einer Innenwandung 11 des Grundkörpers 8 bzw. an einer Außenfläche 12 des Inlays 9 an, so daß eine ausreichende Klemmwirkung erzielt wird, um das Inlay 9 zuverlässig in dem Grundkörper 8 zu fixieren.

Die Erfindung ist nicht auf das dargestellte Ausführungsbeispiel beschränkt und auch für andere Formen von Femurkomponenten 3 anwendbar. Alle Merkmale der Erfindung sind beliebig miteinander kombinierbar.

## Patentansprüche

1. Hüftgelenk-Endoprothese (1) mit einer Gelenkpfanne (2), welche an einer Femurkomponente (3) eine Gelenkkugel (7) aufweist, die mit der Gelenkpfanne (2) eine Gleitpaarung bildet, wobei die Gelenkpfanne (2) einen in einem Beckenknochen verankerbaren Grundkörper (8) und ein damit lösbar verbindbares Inlay (9) umfaßt, wobei zwischen dem Grundkörper (8) und dem Inlay (9) eine Ausgleichshülse (10) vorgesehen ist
**dadurch gekennzeichnet,**
**daß** die Ausgleichshülse (10) aus einem Verbundwerkstoff oder aus dem biokompatiblen Polymer Polyetheretherketon (PEEK) oder aus dem biokompatiblen Polymer Polymethylmethacrylat (PMMA) hergestellt ist.

2. Hüftgelenk-Endoprothese nach Anspruch 1,
**dadurch gekennzeichnet,**
**daß** die Ausgleichshülse (10) aus einem biokompatiblen Verbundwerkstoff hergestellt ist.

3. Hüftgelenk-Endoprothese nach Anspruch 1 oder 2,
**dadurch gekennzeichnet,**
**daß** die Ausgleichshülse (10) eine Beschichtung aufweist.

4. Hüftgelenk-Endoprothese nach einem der Ansprüche 1 bis 3,
**dadurch gekennzeichnet,**
**daß** die Ausgleichshülse (10) konisch geformt ist.

5. Hüftgelenk-Endoprothese nach einem der Ansprüche 1 bis 4,
**dadurch gekennzeichnet,**
**daß** die Ausgleichshülse (10) vollflächig an einer Innenwandung (11) des Grundkörpers (8) und an einer Außenfläche (12) des Inlays (9) anliegt.

6. Hüftgelenk-Endoprothese nach einem der Ansprüche 1 bis 5,
**dadurch gekennzeichnet,**
**daß** das Inlay (9) aus einem keramischen Werkstoff hergestellt ist.

7. Hüftgelenk-Endoprothese nach einem der Ansprüche 1 bis 6,
**dadurch gekennzeichnet,**
**daß** der Grundkörper (8) der Gelenkpfanne (2) aus einem Metall hergestellt ist.

## Claims

1. Hip joint endoprosthesis (1) with an acetabular cup (2), said hip joint endoprosthesis (1) having, on a femoral component (3), a ball (7) which forms a slide pairing with the acetabular cup (2), said acetabular cup (2) comprising a main body (8), which can be anchored in a pelvic bone, and an inlay (9), which can be releasably connected to the main body (8), and a compensating sleeve (10) being provided between the main body (8) and the inlay (9), **characterized in that** the compensating sleeve (10) is made from a composite material or from the biocompatible polymer polyetheretherketone (PEEK) or from the biocompatible polymer polymethyl methacrylate (PMMA).

2. Hip joint endoprosthesis according to Claim 1, **characterized in that** the compensating sleeve (10) is made from a biocompatible composite material.

3. Hip joint endoprosthesis according to Claim 1 or 2, **characterized in that** the compensating sleeve (10) has a coating.

4. Hip joint endoprosthesis according to one of Claims 1 to 3, **characterized in that** the compensating sleeve (10) has a conical shape.

5. Hip joint endoprosthesis according to one of Claims 1 to 4, **characterized in that** the compensating sleeve (10) bears with its entire surface on an inner wall (11) of the main body (8) and on an outer face (12) of the inlay (9).

6. Hip joint endoprosthesis according to one of Claims 1 to 5, **characterized in that** the inlay (9) is made from a ceramic material.

7. Hip joint endoprosthesis according to one of Claims 1 to 6, **characterized in that** the main body (8) of the acetabular cup (2) is made from a metal.

## Revendications

1. Endoprothèse de l'articulation de la hanche (1) avec une cupule d'articulation (2), qui comprend sur un composant de fémur (3), une rotule d'articulation (7) formant un couple de glissement avec la cupule d'articulation (2), la cupule d'articulation (2) comprenant un corps de base (8) pouvant s'ancrer dans un os du bassin et un insert (9) pouvant y être relié de manière amovible, une douille de compensation (10) étant prévue entre le corps de base (8) et l'insert (9),
**caractérisée**
**en ce que** la douille de compensation (10) est fabriquée en un matériau composite, ou en le polymère biocompatible qu'est le polyétheréthercétone (PEEK), ou bien encore en le polymère biocompatible qu'est le polyméthacrylate de méthyle (PMMA).

2. Endoprothèse de l'articulation de la hanche selon la revendication 1,
**caractérisée**
**en ce que** la douille de compensation (10) est fabriquée en un matériau composite biocompatible.

3. Endoprothèse de l'articulation de la hanche selon la revendication 1 ou la revendication 2,
**caractérisée**
**en ce que** la douille de compensation (10) présente un revêtement.

4. Endoprothèse de l'articulation de la hanche selon l'une des revendications 1 à 3,
**caractérisée**
**en ce que** la douille de compensation (10) est d'une forme conique.

5. Endoprothèse de l'articulation de la hanche selon l'une des revendications 1 à 4,
**caractérisée**
**en ce que** la douille de compensation (10) s'appuie selon toute sa surface sur une paroi intérieure (11) du corps de base (8) et sur une surface extérieure (12) de l'insert (9).

6. Endoprothèse de l'articulation de la hanche selon l'une des revendications 1 à 5,
**caractérisée**
**en ce que** l'insert (9) est fabriqué en un matériau céramique.

7. Endoprothèse de l'articulation de la hanche selon l'une des revendications 1 à 6,
**caractérisée**
**en ce que** le corps de base (8) de la cupule d'articulation (2) est fabriquée en un métal.
